# EUROPÄISCHE PATENTSCHRIFT

(11) **EP 1 399 417 B1**
(45) Veröffentlichungstag und Bekanntmachung des Hinweises auf die Patenterteilung: **12.08.2009**
(21) Anmeldenummer: 02776502.3
(22) Anmeldetag: 04.05.2002
(51) Int. Cl.: C07C 303/26, C07C 309/65, C07D 295/02, H01M 6/16, H01M 10/40

(54) **VERFAHREN ZUR HERSTELLUNG VON PERFLUORALKANSULFONSÄUREESTERN**
METHOD FOR PRODUCING PERFLUOROALKANESULFONIC ACID ESTERS
PROCEDE DE PRODUCTION D'ESTERS D'ACIDE PERFLUOROALCANE SULFONIQUES

(30) Priorität: 01.06.2001 DE 10126929; 26.07.2001 DE 10136121
(43) Veröffentlichungstag der Anmeldung: 24.03.2004
(60) Teilanmeldung: 09006446.0
(73) Patentinhaber: Merck Patent GmbH, 64293 Darmstadt (DE)
(72) Erfinder: IGNATYEV, Nikolai, 47058 Duisburg (DE); SCHMIDT, Michael, 64342 Seeheim-Jugenheim (DE); HEIDER, Udo, Winchester SO22 4HZ (GB); SARTORI, Peter, 86919 Utting (DE); KUCHERYNA, Andry, 47053 Duisburg (DE)
(86) Internationale Anmeldenummer: PCT/EP2002/004917
(87) Internationale Veröffentlichungsnummer: WO 2002/098844

(56) Entgegenhaltungen:
- WO-A-01/15258
- WO-A-90/14676
- T. GRAMSTAD, ET AL.: "Perfluoroalkyl derivatives of sulphur. Part IV. Perfluoroalkanesulphonic acids" JOURNAL OF THE CHEMICAL SOCIETY, Nr. 1, 1956, Seiten 173-180, XP002211121 Royal Society of Chemistry, Letchworth, GB in der Anmeldung erwähnt
- T.M. SU, ET AL.: "The solvolysis of highly unreactive substrates using the trifluoromethanesulphonate leaving group" JOURNAL OF THE AMERICAN CHEMICAL SOCIETY, Bd. 91, Nr. 19, 10. September 1969 (1969-09-10), Seiten 5386-5388, XP002211122 American Chemical Society, Washington, DC, US ISSN: 0002-7863
- D.C.R. HOCKLESS, ET AL.: "1-Methyl-phenylphosphirinium triflate: synthesis, structure and reactivity" JOURNAL OF THE CHEMICAL SOCIETY, CHEMICAL COMMUNICATIONS, Nr. 2, 21. Januar 1995 (1995-01-21), Seiten 257-258, XP002211152 Royal Society of Chemistry, Letchworth, GB ISSN: 0022-4936
- D. YANG, ET AL.: "Design of efficient ketone catalysts for epoxidation by using the field effect" JOURNAL OF ORGANIC CHEMISTRY, Bd. 63, Nr. 24, 29. Oktober 1998 (1998-10-29), Seiten 8952-8956, XP002211153 American Chemical Society, Washington, DC, US ISSN: 0022-3263
- J.F. KING, ET AL.: "Betylates. 3. Preparative nucleophilic substitution by way of [2]-, [3]-, and [4]betylates. Stoichiometric phase transfer and substrate-reagent ion-pair (SRIP) reactions of beylates" JOURNAL OF THE AMERICAN CHEMICAL SOCIETY, Bd. 104, Nr. 25, 15. Dezember 1982 (1982-12-15), Seiten 7102-7122, XP002211154 American Chemical Society, Washington, DC, US ISSN: 0002-7863
- S.E. DENMARK, ET AL.: "Catalytic epoxidation of alkenes with oxone" JOURNAL OF ORGANIC CHEMISTRY, Bd. 60, Nr. 5, 10. März 1995 (1995-03-10), Seiten 1391-1407, XP000917828 American Chemical Society, Washington, DC, US ISSN: 0022-3263
- U. CHIACCHIO, ET AL.: "A general synthetic approach to 5-alkyl-2(5H)furanones via 1,3-dipolar cycloaddition" TETRAHEDRON, Bd. 54, Nr. 21, 21. Mai 1998 (1998-05-21), Seiten 5695-5708, XP004118419 Elsevier Science Publishers, Amsterdam, NL ISSN: 0040-4020
- S.E. DENMARK, ET AL.: "Dioxiranes are the active agents in ketone-catalyzed epoxidations with oxone" JOURNAL OF ORGANIC CHEMISTRY, Bd. 62, Nr. 26, 26. Dezember 1997 (1997-12-26), Seiten 8964-8965, XP002211104 American Chemical Society, Washington, DC, US ISSN: 0022-3263
- L. ERNST, ET AL.: "Darstellung und Kristallstrukturen einiger mit N,N'-Dimethylharnstoff verbrückter Diphosphorverbindungen; NMR-Untersuchung einer gamma4P+gamma4P+-Diphosphor- verbindung" CHEMISCHE BERICHTE, Bd. 123, Nr. 1, Januar 1990 (1990-01), Seiten 35-43, XP002211155 Verlag Chemie, Weinheim, DE ISSN: 0009-2940
- D.R. MACFARLANE, ET AL.: "Pyrrolidinium imides: a new family of molten salts and conductive plastic crytal phases" JOURNAL OF PHYSICAL CHEMISTRY B, Bd. 103, Nr. 20, 20. Mai 1999 (1999-05-20), Seiten 4164-4170, XP002211103 American Chemical Society, Washington, DC, US ISSN: 1520-6106
- PATENT ABSTRACTS OF JAPAN vol. 016, no. 582 (E-1300), 22. Dezember 1992 (1992-12-22) -& JP 04 233210 A (MURATA MFG CO LTD), 21. August 1992 (1992-08-21)

## Beschreibung

Die vorliegende Erfindung betrifft ein Verfahren zur Herstellung von Perfluoralkansulfonsäureestern.

Die Verbreitung von tragbaren elektronischen Geräten, wie z.B. Laptop-und Palmtop-Computem, Mobiltelefonen, oder Videokameras und damit auch der Bedarf nach leichten und leistungsfähigen Batterien hat in den letzten Jahren weltweit drastisch zugenommen.

Angesichts dieses sprunghaft gestiegenen Bedarfs nach Batterien und den damit verbundenen ökologischen Problemen kommt der Entwicklung von wiederaufladbaren Batterien mit einer langen Lebensdauer eine stetig wachsende Bedeutung zu.

Lithium-lonen-Batterien und Doppelschichtkondensatoren mit sehr hohen Kapazitäten (sogenannte Super- oder Ultracapacitors) stellen den derzeitigen Stand der Technik dar. In beiden Systemen werden mit LiPF₆ bzw. N(C₂H₅)₄BF₄ derzeit hydrolyseempfindliche und thermisch instabile Substanzen als Leitsalz verwendet. Im Kontakt mit feuchter Luft bzw. mit Restwasser aus den Lösungsmitteln kann schnell HF entstehen. Neben den toxischen Eigenschaften wirkt HF sehr negativ auf das Zyklenverhalten und somit auf die Performance der elektrochemischen Zellen.

Als Alternative wurden Imide, wie das Bis(trifluormethylsulfonyl)imid oder das Bis(pentafluorethylsulfonyl)imid oder Methanide, wie das Tris(trifluormethylsulfonyl)methanid und deren Derivate vorgestellt.

Aber auch quaternäre Ammonium- und Phosphoniumsalze mit Perfluoralkansulfonat-Anionen wurden als Leitsalze für galvanische Zellen entwickelt. Die Synthese dieser Salze ist jedoch relativ aufwendig, da ein Zwischenprodukt, der Trifluormethansulfonsäuremethylester (Methyltriflat), schwer darzustellen ist.

Methyltriflat ist ein starkes Methylierungsreagenz. Es wird in der präparativen Chemie zur Einführung von Methylgruppen genutzt, z.B. bei der Methylierung von heterocyclischen Verbindungen (Yu, Teylor, Tetrahedron Letter, 1999 (36), 6661-6664) oder der Methylierung von Schwefel-organischen Verbindungen (Tsuge, Hatta, Chem. Letter, 1997 (9), 945-946). Methyltriflat ist wesentlich reaktiver als Methyliodid, Dimethylsulfat und Methyltoluensulfonat, die üblicherweise verwendeten Methylierungsreagenzien bei der Synthese von quaternären Ammonium- und Phosphoniumsalzen.

Es gibt verschiedene Synthesewege für das Methyltriflat (Gramstad, J. Chem. Soc., 1956, 173-180 oder Beard, J. Org. Chem., 1973 (21), 3673-3677). Alle beschriebenen Synthesewege eignen sich nicht für eine Übertragung in einen großen Maßstab, da sie entweder sehr toxische Ausgangsmaterialien, wie z.B. Dimethylsulfat, verwenden, die Ausbeuten sehr niedrig sind, das Reaktionsprodukt aufgereinigt werden muß, oder gefährliche Neben- bzw. Abfallprodukte anfallen, wie z.B. Schwefelsäure die mit Dimethylsulfat verunreinigt ist.

Aufgabe der Erfindung war es daher, ein einfaches Verfahren für die Synthese von Perfluoralkansulfonsäurealkylestern und daraus herstellbare Leitsalze zur Verfügung zu stellen.

Die Aufgabe wird gelöst durch ein Verfahren zur Herstellung von Perfluoralkansulfonsäureestern, bei dem Perfluoralkan-sulfonsäureanhydrid in Gegenwart von Perfluoralkansulfonsäure mit Dialkylcarbonat umgesetzt wird, wobei die Reaktion unter wasserfreier Atmosphäre durchgeführt wird, z.B.: 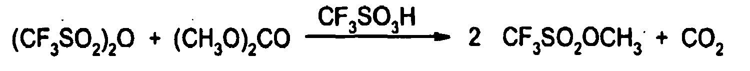 Überraschend wurde gefunden, daß die Umsetzung von Perfluoralkansulfonsäureanhydrid nahezu quantitativ zum Perfluoralkansulfonsäurealkylester erfolgt. Es sind nur katalytische Mengen an Perfluoralkansulfonsäure erforderlich: üblicherweise werden nur 0,01-0,1 mol Perfluoralkansulfonsäure pro 1 mol Perfluoralkansulfonsäureanhydrid benötigt.

Über das efindungsgemäße Verfahren erhält man Perfluoralkansulfonsäurealkylester, die als Alkylierungsreagenzien verwendet werden können. Sie können zur Alkylierung von heterocyclischen Verbindungen oder Phosphor- und Schwefel-organischen Verbindungen verwendet werden oder zur Darstellung von N-Methylaminosäure mit geringer Racematbildung.

Weiterhin können die erhaltenden Perfluoralkansulfonsäureester auch in galvanischen Zellen, primären Batterien, sekundären Batterien, Kondensatoren und/oder Superkondensatoren beispielsweise als Lösungsmittel eingesetzt werden.

Ferner können die erhaltenen Ester weiter umgesetzt werden zu Perfluoralkansulfonsäuresalzen. Vorteilhafterweise ist es nicht unbedingt erforderlich, dass der erfindungsgemäß zuerst erhaltene Ester isoliert werden muss, da nicht umgesetzte Dialkylcarbonate bei der anschließenden Reaktion mit

XR¹R²R³,

wobei
- X: P oder N ist und
- R¹, R², R³: gleich oder verschieden sind, gegebenenfalls durch eine Einfach- oder Doppelbindung direkt miteinander verbunden sind und jeweils einzeln oder gemeinsam die Bedeutung
- eines Wasserstoffs,
- eines Alkylrestes mit 1 bis 16 C-Atomen, der teilweise mit weiteren Gruppen, vorzugsweise F, Cl, N(CₙF₍₂ₙ₊₁ₓ₎Hₓ)₂, O(CₙF₍₂ₙ₊₁₋ₓ₎Hₓ), SO₂(CₙF₍₂ₙ₊₁₋ₓ₎Hₓ), CₙF₍₂ₙ₊₁₋ₓ₎Hₓ mit 1 ≤ n ≤ 6 und 0 ≤ x ≤ 2n+1, ggf. substituiertem Arylrest oder ggf. substituiertem heterocyclischem Rest, substituiert sein kann,
- eines Alkylarylrestes, dessen Alkylengruppe 1 bis 16 C-Atome besitzt und der teilweise mit weiteren Gruppen, vorzugsweise F, Cl, Br, NO₂,
- eines Arylrestes, der teilweise mit weiteren Gruppen, vorzugsweise F, Cl, Br, NO₂, CN, Alkyl, Aryl oder heterocyclischem Rest, substituiert sein kann, oder
- eines heterocyclischen Restes, der teilweise mit weiteren Gruppen, vorzugsweise F, Cl, Br, NO₂, CN, Alkyl, Aryl oder heterocyclischem Rest, substituiert sein kann,CN, Alkyl, Aryl oder heterocyclischem Rest, substituiert sein kann,
besitzen,
wobei ein, zwei oder drei CH₂-Gruppen eines Alkyl-oder Alkylenrestes durch gleiche oder verschiedene Heteroatome, vorzugsweise O, NH oder N(Alkyl) mit 1 bis 6 C-Atomen, ersetzt sein können und wobei R¹, R² und R³ nicht gleichzeitig perfluoriert oder perchloriert sein können,
zu den entsprechenden Perfluoralkansulfonsäuresalzen als Lösungsmittel enthalten sein können. Nach der Umsetzung fällt das Perfluoralkansulfonsäuresalz aus. Der nicht umgesetzte Ester muss lediglich abdestilliert werden, während die restliche Perfluoralkansulfonsäure mit XR¹R²R³ neutralisiert ist bzw. für weitere Umsetzungen genutzt werden kann.

In einer bevorzugten Variante des erfindungsgemäßen Verfahrens wird bei dieser nachfolgenden Umsetzung mit dem Ester eine Verbindung XR¹R²R³ eingesetzt, die ausgewählt ist aus der Gruppe

X(C₂H₅)₃, X(C₃H₇)₃, X(C₄H₉)₃,

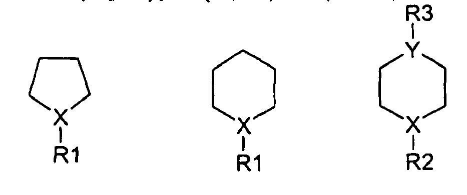

wobei
- X, Y: P oder N ist und
- R¹, R², R³: gegebenenfalls gleich oder verschieden sind, und jeweils einzeln oder gemeinsam die Bedeutung
- eines Wasserstoffes,
- eines Alkylrestes mit 1 bis 16 C-Atomen,
- eines Alkylarylrestes, dessen Alkylengruppe 1 bis 16 C-Atome,
- eines Arylrestes oder
- eines heterocyclischen Restes
besitzen,
wobei ein, zwei oder drei CH₂-Gruppen des Rings und/oder der Alkylreste durch gleiche oder verschiedene Heteroatome, vorzugs-weise O, NH oder N(Alkyl) mit 1 bis 6 C-Atomen, ersetzt sein können und
wobei der Ring und/oder der Alkylrest teilweise mit weiteren Gruppen, vorzugsweise mit F, Cl, N(CₙF₍₂ₙ₊₁₋ₓ₎Hₓ)₂, O(CₙF₍₂ₙ₊₁₋ₓ₎Hₓ), SO₂(CₙF₍₂ₙ₊₁₋ₓ₎Hₓ), CₙF₍₂ₙ₊₁₋ₓ₎Hₓ mit 1 ≤ n ≤ 6 und 0 ≤ x ≤ 2n+1, Alkylaryl, Aryl und/oder heterocyclischem Rest, substituiert sein kann und
wobei der Alkylarylrest, der Arylrest und/oder der heterocyclische Rest teilweise mit weiteren Gruppen, vorzugsweise F, Cl, Br, NO₂, CN, Alkyl, Aryl oder heterocyclischem Rest, substituiert sein kann.

In einer weiteren besonders bevorzugten Variante des erfindungsgemäßen Verfahrens können Perfluoralkansulfonsäurersalze hergestellt werden, indem ein erfindungsgemäß erhaltener Ester mit einer Verbindung ausgewählt aus der folgenden Gruppe umgesetzt wird: 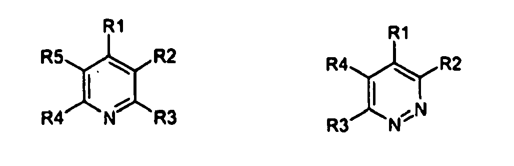 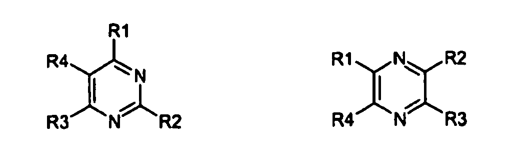 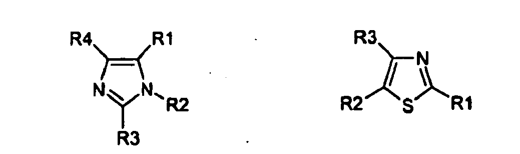 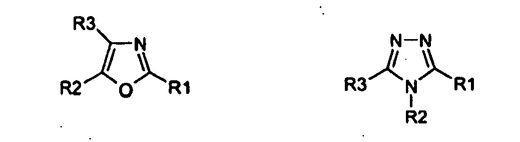 wobei
R¹ bis R⁵ gleich oder verschieden sind, gegebenenfalls durch eine Einfach- oder Doppelbindung direkt miteinander verbunden sind und jeweils einzeln oder gemeinsam die Bedeutung
- eines Wasserstoffs,
- eines Halogens, vorzugsweise Fluor, mit der Maßgabe, dass keine N-Halogen-Bindung vorliegt,
- eines Alkylrests mit 1 bis 8 C-Atomen, der teilweise durch weitere Gruppen, vorzugsweise F, Cl, N(CₙF₍₂ₙ₋₁₋ₓ₎Hₓ)₂, O(CₙF₍₂ₙ₊₁₋ₓ₎Hₓ), SO₂(CₙF₍₂ₙ₊₁₋ₓ₎Hₓ)_{,} CₙF₍₂ₙ₊₁₋ₓ₎Hₓ mit 1 ≤ n ≤ 6 und 0 ≤ x ≤ 2n+1, substituiert sein kann,
- eines Arylrestes
- eines Alkylarylrestes
- eines heterocyclischen Restes
- eines alkylheterocyclischen Restes
besitzen.

Alle erfindungsgemäß hergestellten Perfluoralkansulfonsäureester und insbesondere deren Salze, können in Elektrolyten, galvanischen Zellen, primären und sekundären Batterien, Kondensatoren, Super- bzw. Ultrakondensatoren, beispielsweise als Lösungsmittel oder Leitsalze, eingesetzt werden. Dabei können die Salze sowohl in reiner Form als auch in Form ihrer Mischungen als Leitsalze eingesetzt werden. Es ist auch möglich, die Salze gemeinsam mit weiteren, dem Fachmann bekannten Salzen als Leitsalz zu verwenden.

Die erfindungsgemäß hergestellten Perfluoralkansulfonsäurerester, insbesondere die Salze können in flüssigen, gelartigen, polymeren oder festen Elektrolyten verwendet werden. Hierzu können Gemische enthaltend die Leitsalze sowie geeignete Polymeren und/oder geeignete Lösungsmittel eingesetzt werden. Ein Gemisch im Sinne der vorliegenden Erfindung umfasst reine Mischungen der Komponenten, Mischungen, in denen das oder die Salze in einem Polymeren oder Gel eingeschlossen sind sowie Mischungen, in denen zwischen dem oder den Salzen und einem Polymeren oder Gel chemische und/oder physikalische Bindungen bestehen. Im Fall eines gelartigen Elektrolyten enthält das Gemisch vorzugsweise neben dem oder den Salzen und dem Polymer ein geeignetes Lösungsmittel.

Als Lösungsmittel für flüssige oder gelartige Elektrolyte werden besonders bevorzugt aprotische Lösungsmittel oder deren Gemische eingesetzt, die zur Anwendung in einer primären oder sekundären Batterie, einem Kondensator, einem Superkondensator oder einer galvanischen Zelle geeignet sind, zum Beispiel Carbonate, Ester, Ether, Sulfolane oder Nitrile wie beispielsweise Dimethylcarbonat, Diethylcarbonat, Butylencarbonat, Propylencarbonat, Ethylencarbonat, Ethylmethylcarbonat, Methylpropylcarbonat, 1,2-Dimethoxyethan, 1,2-Diethoxyethan, Methylacetat, γ-Butyrolacton, Ethylacetat, Methylpropionat, Ethylpropionat, Methylbutyrat, Ethylbutyrat, Dimethylsulfoxid, Dioxolan, Sulfolan, Acetonitril, Acrylnitril, Tetrahydrofuran, 2-Methyltetrahydrofuran oder deren Gemische.

Als Polymere für polymere oder gelartige Elektrolyte werden vorzugsweise Homopolymere oder Copolymere von Acrylnitril, Vinylidendifluorid, Methyl(meth)acrylat, Tetrahydrofuran, Ethylenoxid, Siloxan, Phosphazen oder eine Mischung aus wenigstens zwei der vorstehend genannten Homopolymeren und/oder Copolymeren eingesetzt, wobei die Polymere zumindest teilweise vernetzt sein können.

Die so erhaltenen Elektrolyte eignen sich zum Einsatz in primären Batterien, sekundären Batterien, Kondensatoren, Super- bzw. Ultrakondensatoren und galvanischen Zellen.

Die im folgenden angeführten Beispiele für den erfindungsgemäßen Gegenstand dienen lediglich der Erläuterung und engen die vorliegende Erfindung keineswegs in irgendeiner Weise ein. Im übrigen ist die beschriebene Erfindung im gesamten beanspruchten Bereich ausführbar.

Alle NMR-Spektren wurden auf einem Bruker Spektrometer WP 80 SY gemessen.

### Beispiele

### Beispiel 1

### Darstellung von Methyltrifluormethansulfonat (Methyltriflat)

In einem 1 I-Rundhalskolben werden 646 g (2,29 mol) Trifluormethansulfonsäureanhydrid und 36 g (0,24 mol) Trifluormethansulfonsäure vorgelegt. Unter ständigem Rühren und Rückflußkühlung werden 206 g (2,29 mol) Dimethylcarbonat bei Raumtemperatur zugegeben. Innerhalb von 10 min erwärmt sich die Lösung auf 50-60°C und wird anschließend noch eine Stunde bei dieser Temperatur weiter gerührt. Anschließend wird die Lösung mit einem Ölbad auf 100-110°C erwärmt und weitere 2 Stunden gerührt. Es werden nach einer Destillation 733 g Methyltrifluormethansulfonat mit einer Reinheit über 99% isoliert (Siedebereich: 98-99°C, Ausbeute: 97,7%).

¹⁹F und ¹H-NMR sind identisch mit den Literaturdaten (Paquette, Encyclopedia of Reagents for Organic Synthesis, 1995, 3617-3622).

Zu dem Rückstand werden 731 g (2,59 mol) Trifluormethansulfonsäureanhydrid und 232 g (2,58 mol) Dimethylcarbonat gegeben und der oben beschriebene Prozeß wiederholt. Es werden 837 g Methyltrifluormethansulfonat mit einer Reinheit über 99% isoliert (Ausbeute: 99,1%).

Der Prozess kann mehrmals wiederholt werden.

### Beispiel 2

### Darstellung von Methylpentafluorethansulfonat

In einem 10 ml-Rundhalskolben werden 5,74 g (15,0 mmol) Pentafluorethansulfonsäureanhydrid und 0,31 g (1,55 mmol) Pentafluorethansulfonsäure vorgelegt. Unter ständigem Rühren und Rückflußkühlung werden 1,35 g (15,0 mmol) Dimethylcarbonat bei Raumtemperatur zugegeben. Die Lösung wird eine Stunde bei einer Temperatur von 60°C und anschließend bei 110°C 3 Stunden weiter gerührt. Es werden nach einer Destillation 5,41 g Methylpentafluorethansulfonat isoliert (Siedebereich: 114-115°C, Ausbeute: 84,1%).

NMR ¹⁹F, ppm: (Lösungsmittel: CDCl₃; Standard: CCl₃F): -80,44 s (CF₃); -115,34 s (CF₂)
NMR ¹H, ppm: (Lösungsmittel: CDCl₃; Standard: TMS): 4,23 s (CH₃)

### Beispiel 3

### Darstellung von N-Methyl-N-triethylammoniumtrifluormethansulfonat

Bei Raumtemperatur wird eine Lösung aus 8,35 g (82,7 mmol) Triethylamin in 150 cm³ trockenem Hexan vorgelegt. Unter ständigem Rühren werden innerhalb von 10 min 13,56 g (82,7 mmol) Methyltriflat, hergestellt wie in Beispiel 1, bei Raumtemperatur zugegeben. Die Lösung erwärmt sich und wird eine halbe Stunde weiter gerührt. Dabei wird die Lösung wieder auf Raumtemperatur gebracht. Ein weißer Niederschlag wird abfiltriert und mit Hexan gewaschen. Das Hexan-Filtrat kann für eine weitere Umsetzung genutzt werden. Nach der Trocknung im Vakuum bei 60°C werden 21,81 g eines weißen feinkristallinen Materials werden isoliert (Ausbeute: 99,5%).

NMR ¹⁹F, ppm: (Lösungsmittel: Acetonitril-D₃; Standard: CCl₃F): -78,04 s (CF₃SO₃⁻)
NMR ¹H, ppm: (Lösungsmittel: Acetonitril-D₃; Standard: TMS): 1,25 tm (3CH₃); 2,86 s (CH₃); 3,26 q (3CH₂); J³_{H,H}=7,3 Hz

¹H-NMR-Daten entsprechen den Literaturdaten (R. Weiß, K.-G. Wagner, M. Hertel, Chem. Ber. 117 (1984) S.1965-1972)

### Beispiel 4

### Darstellung von Methyl(triethyl)phosphoniumtrifluormethansulfonat

Bei Raumtemperatur wird eine Lösung aus 8,05 g (68,2 mmol) Triethylphosphin in 150 cm³ trockenem Hexan vorgelegt. Unter ständigem Rühren werden innerhalb von 10 min 11,19 g (68,2 mmol) Methyltriflat, hergestellt wie in Beispiel 1, bei Raumtemperatur zugegeben. Die Lösung erwärmt sich und wird eine halbe Stunde weiter gerührt. Dabei wird die Lösung wieder auf Raumtemperatur gebracht. Ein weißer Niederschlag wird abfiltriert und mit Hexan gewaschen. Das Hexan-Filtrat kann für eine weitere Umsetzung genutzt werden. Nach der Trocknung im Vakuum bei 60°C werden 19,02 g eines weißen feinkristallinen Materials isoliert (Schmelzpunkt: 103-104°C, Ausbeute: 98,9%).

NMR ¹⁹F, ppm (Lösungsmittel: Aceton-D₆; Standard: CCl₃F): -77,82 s (CF₃SO₃⁻)
NMR ¹H, ppm (Lösungsmittel: Aceton-D₆; Standard: TMS): 1,30 dt (3CH₃); 1,95 d (CH₃); 2,40 dq (3CH₂); J³_{H,H}=7,7 Hz; J²_{P,H}=13,7 Hz; J²_{P,H}=13,8 Hz; J³_{P,H}=18,8 Hz

| Elementaranalyse: | | | |
|---|---|---|---|
| gefunden: | 33,72% C, | 6,57% H, | 11,14% S |
| berechnet: | 34,04% C, | 6,43% H, | 11,36% S ((C₂H₅)₃PCH₃⁺ CF₃SO₃⁻) |

### Beispiel 5

### Darstellung von 1,3-Dimethylimidazoliumtrifluormethansulfonat

Zu 6,67 g (81,2 mmol) 1-Methylimidazol werden in einem Rundkolben unter Rühren und Eisbad-Kühlung 13,70 g (83,5 mmol) Methyltriflat innerhalb von 10 Minuten zugetropft. Das Reaktionsgemisch erwärmt sich und wird unter Rückflußkühlung 1 Stunde bei 70-75°C gerührt. Der Überschuss an Methyltriflat wird im Vakuum bei 60°C entfernt. 20,00 g eines weißen Pulvers werden isoliert.

NMR ¹⁹F, ppm (Lösungsmittel: Acetonitril-D₃; Standard: CCl₃F): -78,14 s (CF₃SO₃⁻)
NMR ¹H, ppm (Lösungsmittel: Acetonitril-D₃; Standard: TMS): 3,82 s (2CH₃); 7,35 d (2H); 8,53 t (1 H); .J⁴_{H;H} = 1,6 Hz

¹H-NMR-Daten entsprechen den Literaturdaten (U. Zollner, Tetrahedron, 44, No.24 (1988), S.7413-7426)

### Beispiel 6

### Darstellung von N,N-Dimethylpyrrolidiniumtrifluormethansulfonat

Bei Raumtemperatur wird eine Lösung aus 6,83g (80,2mmol) N-Methylpyrrolidin in 150 cm³ trockenem Hexan vorgelegt. Unter ständigem Rühren werden innerhalb von 10min 13,12g (80,2mmol) Methyltriflat, hergestellt wie in Beispiel 1, bei Raumtemperatur zugegeben, wobei sich die Lösung erwärmt. Nach einer halben Stunde wird die Lösung wieder auf Raumtemperatur gebracht. Der aufgefallene weiße Niederschlag wird abfiltriert mit Hexan gewaschen und im Vakuum bei 60°C getrocknet. Es werden 16,61 g eines weißen feinkristallinen Materials isoliert (Schmelzpunkt (mit Zersetzung): 308-310°C, Ausbeute 98,3%).

NMR ¹⁹F, ppm (Lösungsmittel: Acetonitril-D₃; Standard: CCl₃F): -78,00 s (CF₃SO₃⁻)
NMR ¹H, ppm (Lösungsmittel: Acetonitril-D₃; Standard: TMS): 2,17 m (2H); 3,07 s (CH₃); 3,45 m (2H)

| Elementaranalyse nach Umkristallisation aus Methanol: | | | | |
|---|---|---|---|---|
| gefunden: | 33,66% C, | 5,68% H, | 5,60% N, | 13,06% S |
| berechnet: | 33,73% C, | 5,66% H, | 5,62% N, | 12,86% S (C₇H₁₄ F₃NO₃S) |

### Beispiel 7

### Darstellung von N,N-Dimethylpiperidiniumtrifluormethansulfonat

Bei Raumtemperatur wird eine Lösung aus 7,76g (78,2mmol) N-Methylpiperidin in 150 cm³ trockenem Hexan vorgelegt. Unter ständigem Rühren werden innerhalb von 10min 12,83g (78,2mmol) Methyltriflat, hergestellt wie in Beispiel 1, bei Raumtemperatur zugegeben, wobei sich die Lösung erwärmt. Nach einer halben Stunde wird die Lösung wieder auf Raumtemperatur gebracht. Der aufgefallene weiße Niederschlag wird abfiltriert mit Hexan gewaschen und im Vakuum bei 80°C getrocknet. Es werden 19,72g eines weißen feinkristallinen Materials isoliert (Schmelzpunkt nach Umkristallisation aus Methanol: 255-256°C, Ausbeute 95,8%).

NMR ¹⁹F, ppm (Lösungsmittel: Acetonitril-D₃; Standard: CCl₃F): -78,06 s (CF₃SO₃⁻)
NMR ¹H, ppm (Lösungsmittel: Acetonitril-D₃; Standard: TMS): 1,79 m (3CH₂); 3,02 s (2CH₃); 3,27 m (2CH₂)

| Elementaranalyse nach Umkristallisation aus Methanol: | | | | |
|---|---|---|---|---|
| gefunden: | 36,44% C, | 6,07% H, | 5,31% N, | 12,20% S |
| berechnet: | 36,50% C, | 6,13% H, | 5,32% N, | 12,18% S (C₈H₁₆ F₃NO₃S) |

## Patentansprüche

1. Verfahren zur Herstellung von Perfluoralkansulfonsäureestern, bei dem Perfluoralkansulfonsäureanhydrid in Gegenwart von Perfluoralkansulfonsäure mit Dialkylcarbonat umgesetzt wird.

2. Verfahren nach Anspruch 1,
**dadurch gekennzeichnet, dass** das Dialkylcarbonat ausgewählt ist aus der Gruppe: Dimethylcarbonat, Diethylcarbonat, Dipropylcarbonat, Dibutylcarbonat oder deren Mischungen.

3. Verfahren zur Herstellung von Perfluoralkansulfonsäuresalzen
**dadurch gekennzeichnet, dass** der nach Anspruch 1 hergestellte Perfluoralkansulfonsäurealkylester mit einer Verbindung gemäß der Formel
XR¹R²R³,
wobei
X P oder N ist und
R¹, R², R³ gleich oder verschieden sind, gegebenenfalls durch eine Einfach- oder Doppelbindung direkt miteinander verbunden sind und jeweils einzeln oder gemeinsam die Bedeutung
- eines Wasserstoffs,
- eines Alkylrestes mit 1 bis 16 C-Atomen, der teilweise mit weiteren Gruppen, vorzugsweise F, Cl, N(CₙF₍₂ₙ₊₁₊ₓ)Hₓ)₂, O(CₙF₍₂ₙ₊₁₋ₓ₎Hₓ), SO₂(CₙF₍₂ₙ₊₁₋ₓ₎Hₓ), CₙF₍₂ₙ₊₁₋ₓ₎Hₓ mit 1 ≤ n ≤ 6 und 0 ≤ x ≤ 2n+1, ggf. substituiertem Arylrest oder ggf. substituiertem heterocyclischem Rest, substituiert sein kann,
- eines Alkylarylrestes, dessen Alkylengruppe 1 bis 16 C-Atome besitzt und der teilweise mit weiteren Gruppen, vorzugsweise F, Cl, Br, NO₂, CN, Alkyl, Aryl oder heterocyclischem Rest, substituiert sein kann,
- eines Arylrestes, der teilweise mit weiteren Gruppen, vorzugsweise F, Cl, Br, NO₂, CN, Alkyl, Aryl oder heterocyclischem Rest, substituiert sein kann, oder
- eines Heteroarylrestes, der teilweise mit weiteren Gruppen, vorzugsweise F, Cl, Br, NO₂, CN, Alkyl, Aryl oder heterocyclischem Rest, substituiert sein kann,
besitzen,
wobei ein, zwei oder drei CH₂-Gruppen eines Alkyl-oder Alkylenrestes durch gleiche oder verschiedene Heteroatome, vorzugsweise O, NH oder N(Alkyl) mit 1 bis 6 C-Atomen, ersetzt sein können
und wobei nicht alle R¹, R² und R³ gleichzeitig perfluoriert oder perchloriert sein können,
umgesetzt wird.

4. Verfahren nach Anspruch 3, **dadurch gekennzeichnet, dass** XR¹R²R³ ausgewählt ist aus der Gruppe:
X(C₂H₅)₃, X(C₃H₇)₃, X(C₄H₉)₃, 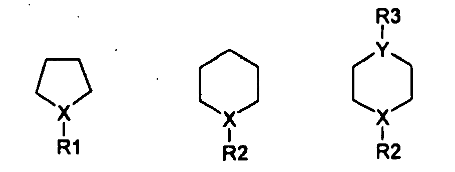
wobei
X, Y P oder N ist und
R¹, R², R³ gegebenenfalls gleich oder verschieden sind, und jeweils einzeln oder gemeinsam die Bedeutung
- eines Wasserstoffes,
- eines Alkylrestes mit 1 bis 16 C-Atomen,
- eines Alkylarylrestes, dessen Alkylengruppe 1 bis 16 C-Atome,
- eines Arylrestes oder
- eines heterocyclischen Restes
besitzen,
wobei ein, zwei oder drei CH₂-Gruppen des Rings und/oder der Alkylreste durch gleiche oder verschiedene Heteroatome, vorzugsweise O, NH oder N(Alkyl) mit 1 bis 6 C-Atomen, ersetzt sein können und
wobei der Ring und/oder der Alkylrest teilweise mit weiteren Gruppen, vorzugsweise mit F, Cl, N(CₙF₍₂ₙ₊₁₋ₓ₎Hₓ)₂, O(CₙF₍₂ₙ₊₁₋ₓ₎Hₓ), SO₂(CₙF₍₂ₙ₊₁₋ₓ₎Hₓ), CₙF₍₂ₙ₊₁₋ₓ₎Hₓ mit 1 ≤ n ≤ 6 und 0 ≤ x ≤ 2n+1, Alkylaryl, Aryl und/oder heterocyclischem Rest, substituiert sein können und
wobei der Alkylarylrest, der Arylrest und/oder der heterocyclische Rest teilweise mit weiteren
Gruppen, vorzugsweise F, Cl, Br, NO₂, CN, Alkyl, Aryl oder heterocyclischem Rest, substituiert sein kann.

5. Verfahren zur Herstellung von Perfluoralkansulfonsäuresalzen nach Anspruch 3
**dadurch gekennzeichnet, dass** der nach Anspruch 1 hergestellte Perfluoralkansulfonsäureester mit einer Verbindung ausgewählt aus der folgenden Gruppe umgesetzt wird: 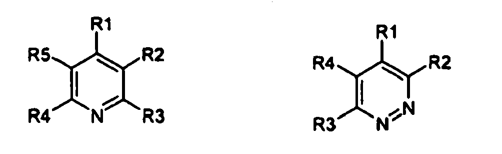 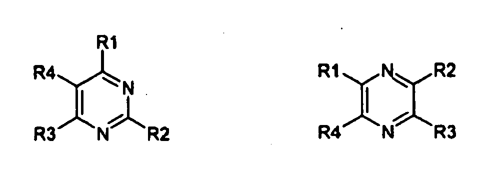 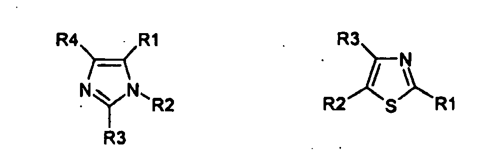 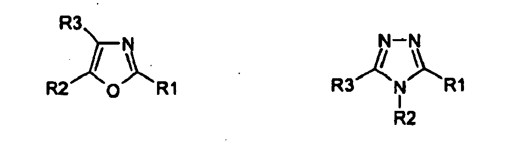 wobei
R¹ bis R⁵ gleich oder verschieden sind, gegebenenfalls durch eine Einfach- oder Doppelbindung direkt miteinander verbunden sind und jeweils einzeln oder gemeinsam die Bedeutung
- eines Wasserstoffs,
- eines Halogens, vorzugsweise Fluor, mit der Maßgabe, dass keine N-Halogen-Bindung vorliegt,
- eines Alkylrests mit 1 bis 8 C-Atomen, der teilweise mit weiteren Gruppen, vorzugsweise F, Cl, N(CₙF₍₂ₙ₋₁₋ₓ₎Hₓ)₂, O(CₙF₍₂ₙ₊₁₋ₓ₎Hₓ), SO₂(CₙF₍₂ₙ₊₁₋ₓ₎Hₓ), CₙF₍₂ₙ₊₁₋ₓ₎Hₓ mit 1 ≤ n ≤ 6 und 0 ≤ x ≤ 2n+1, substituiert sein kann,
- eines Arylrestes
- eines Alkylarylrestes
- eines heterocyclischen Restes
- eines alkylheterocyclischen Restes
besitzen.

## Claims

1. Process for the preparation of perfluoroalkanesulfonic acid esters in which perfluoroalkanesulfonic anhydride is reacted with dialkyl carbonate in the presence of perfluoroalkanesulfonic acid.

2. Process according to Claim 1,
**characterised in that** the dialkyl carbonate is selected from the group: dimethyl carbonate, diethyl carbonate, dipropyl carbonate, dibutyl carbonate or mixtures thereof.

3. Process for the preparation of perfluoroalkanesulfonic acid salts,
**characterised in that** the alkyl perfluoroalkanesulfonate prepared according to Claim 1 is reacted with a compound of the formula
XR¹R²R³,
where
X is P or N, and
R¹, R², R³ are identical or different, are optionally bonded directly to one another via a single or double bond and each, individually or together, have the mean-ing of
- a hydrogen,
- an alkyl radical having 1 to 16 C atoms, which may be partially substituted by further groups, preferably F, Cl, N(CₙF₍₂ₙ₊₁₋ₓ₎Hₓ)₂, O(CₙF₍₂ₙ₊₁₋ₓ₎Hₓ), SO₂(CₙF₍₂ₙ₊₁₋ₓ₎Hₓ), CₙF₍₂ₙ₊₁₋ₓ₎Hₓ,where 1 ≤ n ≤ 6 and 0 ≤ x ≤ 2n+1, an optionally substituted aryl radical or an optionally substituted heterocyclic radical,
- an alkylaryl radical whose alkylene group has 1 to 16 C atoms and which may be partially substituted by further groups, preferably F, Cl, Br, NO₂, CN, alkyl, aryl or a heterocyclic radical,
- an aryl radical, which may be partially substituted by further groups, preferably F, Cl, Br, NO₂, CN, alkyl, aryl or a heterocyclic radical, or
- a heteroaryl radical, which may be partially substituted by further groups, preferably F, Cl, Br, NO₂, CN, alkyl, aryl or a heterocyclic radical,
where one, two or three CH₂ groups of an alkyl or alkylene radical may be replaced by identical or different heteroatoms, preferably O, NH or N(alkyl) having 1 to 6 C atoms,
and where R¹, R² and R³ cannot all simultaneously be perfluorinated or perchlorinated.

4. Process according to Claim 3, **characterised in that** XR¹R²R³ is selected from the group:
X(C₂H₅)₃, X(C₃H₇)₃, X(C₄H₉)₃, 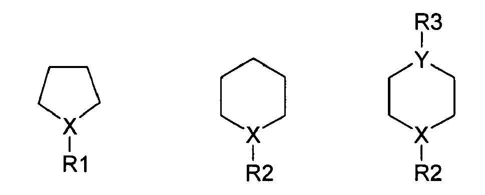
where
X, Y are P or N, and
R¹, R², R³ are optionally identical or different and each, individually or together, have the meaning of
- a hydrogen,
- an alkyl radical having 1 to 16 C atoms,
- an alkylaryl radical whose alkylene group has 1 to 16 C atoms,
- an aryl radical or
- a heterocyclic radical,
where one, two or three CH₂ groups in the ring and/or the alkyl radicals may be replaced by identical or different heteroatoms, preferably O, NH or N(alkyl) having 1 to 6 C atoms, and
where the ring and/or the alkyl radical may be partially substituted by further groups, preferably by F, Cl, N(CₙF₍₂ₙ₊₁₋ₓ₎Hₓ)₂, O(CₙF₍₂ₙ₊₁₋ₓ₎Hₓ),
SO₂(CₙF₍₂ₙ₊₁₋ₓ₎Hₓ), CₙF₍₂ₙ₊₁₋ₓ₎Hₓ, where 1 ≤ n ≤ 6 and 0 ≤ x ≤ 2n+1, alkylaryl, aryl and/or a heterocyclic radical, and
where the alkylaryl radical, the aryl radical and/or the heterocyclic radical may be partially substituted by further groups, preferably F, Cl, Br, NO₂, CN, alkyl, aryl or a heterocyclic radical.

5. Process for the preparation of perfluoroalkanesulfonic acid salts according to Claim 3,
**characterised in that** the perfluoroalkanesulfonic acid ester prepared according to Claim 1 is reacted with a compound selected from the following group: 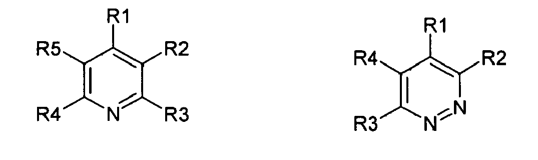 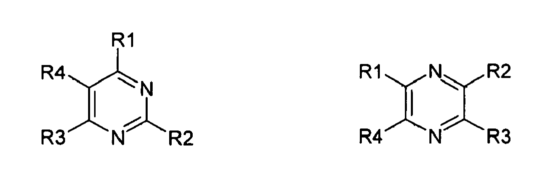 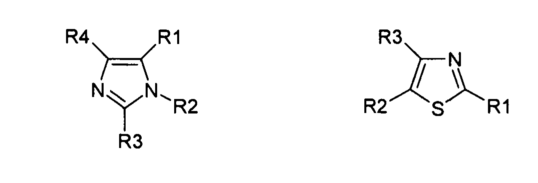 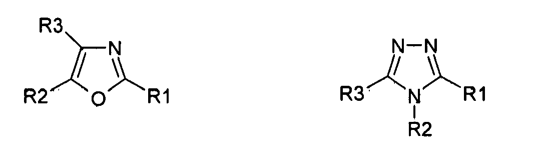 where
R¹ to R⁵ are identical or different, are optionally bonded directly to one another via a single or double bond and each, individually or together, have the meaning of
- a hydrogen,
- a halogen, preferably fluorine,
with the proviso that no N-halogen bond is present,
- an alkyl radical having 1 to 8 C atoms, which may be partially substituted by further groups, preferably F, Cl, N(CₙF₍₂ₙ₊₁₋ₓ₎Hₓ)₂, O(CₙF₍₂ₙ₊₁₋ₓ₎Hₓ), SO₂(CₙF₍₂ₙ₊₁₋ₓ₎Hₓ), CₙF₍₂ₙ₊₁₋ₓ₎Hₓ, where 1 ≤ n ≤ 6 and 0 ≤ x ≤ 2n+1,
- an aryl radical,
- an alkylaryl radical,
- a heterocyclic radical,
- an alkylheterocyclic radical.

## Revendications

1. Procédé de préparation d'esters de l'acide perfluoroalcanesulfonique dans lequel de l'anhydride perfluoroalcanesulfonique est réagi avec du carbonate de dialkyle en présence d'acide perfluoroalcanesulfonique.

2. Procédé selon la revendication 1,
**caractérisé en ce que** le carbonate de dialkyle est choisi parmi le groupe constitué par : le carbonate de diméthyle, le carbonate de diéthyle, le carbonate de dipropyle, le carbonate de dibutyle ou des mélanges de ceux-ci.

3. Procédé de préparation de sels d'acide perfluoroalcanesulfonique,
**caractérisé en ce que** le perfluoroalcanesulfonate d'alkyle préparé selon la revendication 1 est réagi avec un composé de formule
XR¹R²R³,
dans laquelle
X est P ou N, et
R¹, R², R³ sont identiques ou différents, sont éventuellement liés directement l'un à l'autre par une liaison simple ou double et chacun, individuellement ou ensemble, ont la signification de
- un hydrogène,
- un radical alkyle ayant de 1 à 16 atomes de C, pouvant être partiellement substitué par des groupements supplémentaires, préférablement F, CI, N(CₙF₍₂ₙ₊₁₋ₓ₎Hₓ)₂, O(CₙF₍₂ₙ₊₁₋ₓ₎Hₓ), SO₂(CₙF₍₂ₙ₊₁₋ₓ₎Hₓ), CₙF₍₂ₙ₋₁₋ₓ₎Hₓ, où 1 ≤ n ≤ 6 et 0 ≤ x ≤ 2n+1, un radical aryle éventuellement substitué ou un radical hétérocyclique éventuellement substitué,
- un radical alkylaryle dont le groupement alkylène a de 1 à 16 atomes de C et pouvant être partiellement substitué par des groupements supplémentaires, préférablement F, CI, Br, NO₂, CN, alkyle, aryle ou un radical hétérocyclique,
- un radical aryle, pouvant être partiellement substitué par des groupements supplémentaires, préférablement F, Cl, Br, NO₂, CN, alkyle, aryle ou un radical hétérocyclique, ou
- un radical hétéroaryle, pouvant être partiellement substitué par des groupements supplémentaires, préférablement F, CI, Br, NO₂, CN, alkyle, aryle ou un radical hétérocyclique,
où un, deux ou trois groupements CH₂ d'un radical alkyle ou alkylène peuvent être remplacés par des hétéroatomes identiques ou différents, préférablement O, NH ou N(alkyl) ayant de 1 à 6 atomes de C,
et où R¹, R² et R³ ne peuvent être tous simultanément perfluorés ou perchlorés.

4. Procédé selon la revendication 3, **caractérisé en ce que** XR¹R²R³ est choisi parmi le groupe constitué par :
X(C₂H₅)₃, X(C₃H₇)₃, X(C₄H₉)₃, 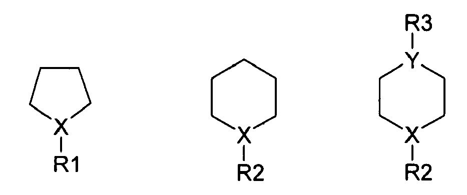
où
X, Y sont P ou N, et
R¹, R², R³ sont éventuellement identiques ou différents et chacun, individuellement ou ensemble, ont la signification de
- un hydrogène,
- un radical alkyle ayant de 1 à 16 atomes de C,
- un radical alkylaryle dont le groupement alkylène a de 1 à 16 atomes de C,
- un radical aryle ou
- un radical hétérocyclique,
où un, deux ou trois groupements CH₂ dans le cycle et/ou les radicaux alkyle peuvent être remplacés par des hétéroatomes identiques ou différents, préférablement O, NH ou N(alkyl) ayant de 1 à 6 atomes de C, et
où le cycle et/ou le radical alkyle peuvent être partiellement substitués par des groupements supplémentaires, préférablement par F, Cl, N(CₙF₍₂ₙ₊₁₋ₓ₎Hₓ)₂, O(CₙF₍₂ₙ₊₁₋ₓ₎Hₓ), SO₂(CₙF₍₂ₙ₊₁₋ₓ₎Hₓ), CₙF₍₂ₙ₊₁₋ₓ₎Hₓ, où 1 ≤ n ≤ 6 et 0 ≤ x ≤ 2n+1, alkylaryle, aryle et/ou un radical hétérocyclique, et
où le radical alkylaryle, le radical aryle et/ou le radical hétérocyclique peuvent être partiellement substitués par des groupements supplémentaires, préférablement F, CI, Br, NO₂, CN, alkyle, aryle ou un radical hétérocyclique.

5. Procédé de préparation de sels d'acide perfluoroalcanesulfonique selon la revendication 3,
**caractérisé en ce que** l'ester de l'acide perfluoroalcanesulfonique préparé selon la revendication 1 est réagi avec un composé choisi parmi le groupe constitué par : 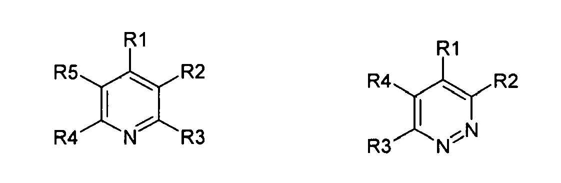 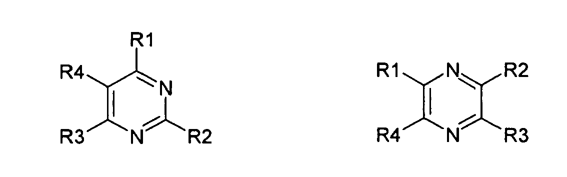 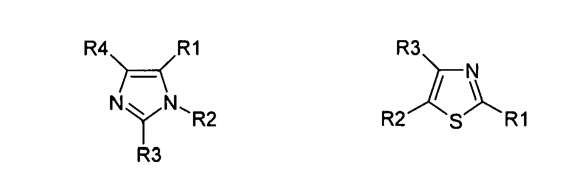 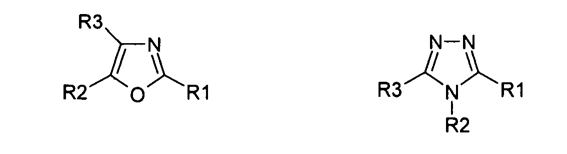 où
R¹ à R⁵ sont identiques ou différents, sont éventuellement liés directement l'un à l'autre par une liaison simple ou double et chacun, individuellement ou ensemble, ont la signification de
- un hydrogène,
- un halogène, préférablement fluor, à condition qu'aucune liaison N-halogène ne soit présente,
- un radical alkyle ayant 1 à 8 atomes de C, pouvant être partiellement substitué par des groupements supplémentaires, préférablement F, Cl, N(CₙF₍₂ₙ₊₁₋ₓ₎Hₓ)₂, O(CₙF₍₂ₙ₊₁₋ₓ₎Hₓ), SO₂(CₙF₍₂ₙ₊₁₋ₓ₎Hₓ), CₙF₍₂ₙ₊₁₋ₓ₎Hₓ,
- un radical aryle,
- un radical alkylaryle,
- un radical hétérocyclique,
- un radical alkylhétérocyclique.
